# EUROPÄISCHE PATENTANMELDUNG

(11) **EP 1 990 007 A1**
(43) Veröffentlichungstag der Anmeldung: **12.11.2008**
(21) Anmeldenummer: 08007804.1
(22) Anmeldetag: 23.04.2008
(51) Int. Cl.: A61B 6/12, A61B 19/00, F16B 2/16, G05G 1/12

(54) **Lokalisationseinheit für eine chirurgische Röntgendiagnostikeinrichtung mit Sterilabdeckfolie**

(30) Priorität: 05.05.2007 DE 102007021182
(71) Anmelder: Ziehm Imaging GmbH, 90451 Nürnberg (DE)
(72) Erfinder: Dehler, Jürgen, 91301 Forchheim (DE)

(57) **Zusammenfassung**

Die Erfindung betrifft eine Lokalisationseinheit für eine mit einer Sterilabdeckfolie abgedeckten Röntgendiagnostikeinrichtung. Die Lokalisationseinheit ist sterilisierbar und weist einen Adapter auf, der ein Mittel zur Durchstoßung der Sterilabdeckfolie und ein Mittel zum temporären Fixieren der Sterilabdeckfolie am Adapter enthält. Der Adapter ist in vorbestimmten Aufnahmen lösbar am Gehäuse der Röntgendiagnostikeinrichtung gehalten und reproduzierbar zu diesem positionierbar, wobei das Mittel zum temporären Fixieren der Sterilabdeckfolie am Gehäuse der Röntgendiagnostikeinrichtung aus einer elastisch verformbaren umlaufenden Dichtung besteht, die beim Positionieren des Adapters eine Kraft auf die Sterilabdeckfolie in Richtung senkrecht zu der Oberfläche des Gehäuses erzeugt.

## Beschreibung

Die Erfindung betrifft eine Lokalisationseinheit für eine mit einer Sterilabdeckfolie abgedeckten Röntgendiagnostikeinrichtung.

Medizinische Eingriffe an lebenden Objekten werden zunehmend mittels Navigationsunterstützung durchgeführt. Darunter versteht man die mittels eines Lageerfassungssystems unterstützte Führung eines Instrumentes relativ zu einem in Behandlung stehenden Gewebebereichs des Objektes. Von besonderem Interesse ist die Navigation in Bereichen, die sich einer optischen Kontrolle des Operateurs entziehen, weil das Instrument beispielsweise in das Innere des Objektes eingeführt wurde. Hierzu wird die Führung des Instrumentes, beispielsweise eines Katheders, in einem virtuellen 3D-Volumen vorgenommen, welches mittels eines bildgebenden Verfahrens vor oder während der Operation erzeugt wurde. Eine häufige Anwendung ist, mit Hilfe einer Röntgendiagnostikeinrichtung eine Reihe von 2D-Projektionsaufnahmen bekannter Projektionsgeometrie zu erzeugen und aus diesen 2D-Aufnahmen einen 3D-Volumendatensatz zu erzeugen. Der Volumendatensatz wird an ein Navigationssystem übergeben, das über ein Lageerfassungssystem für von diesem erfaßbaren Marken verfügt. Um eine Navigation mit hoher Genauigkeit möglich zu machen, wird das Koordinatensystem des Lageerfassungssystem mit dem Koordinatensystem des 3D-Volumendatensatzes abgeglichen. Dieser Vorgang wird üblicherweise "Registrierung" genannt.

Aus der Patentliteratur sind Röntgendiagnostikeinrichtungen bekannt, bei denen Einrichtungsteile mit von einem Lageerfassungssystem erfaßbaren Marken versehen sind.

Aus der deutschen Offenlegungsschrift DE 196 40 993 A1 ist ein von einer Sterilisierungshülle umschlossenes medizinisches Therapie- und/oder Diagnosegerät bekannt, bei dem ein sterilisierbares und betriebsmäßig lösbares Aufsetzteil oberhalb der Sterilisierungshülle in eine Betriebsstellung an dem medizinischen Therapie- und/oder Diagnostikgerät angekoppelt wird, wodurch eine definierte Lagebeziehung zwischen dem Therapie- und/oder Diagnosegerät und dem Aufsetzteil hergestellt werden soll. Dabei ist vorgesehen, daß Paßstifte beim Aufsetzen des Aufsetzteils die Sterilisierungshülle durchstoßen und in Paßstiftausnehmungen aufgenommen werden. Es ist vorgesehen, das Aufsetzteil mittels einer Rändelschraube an dem Therapie- und/oder Diagnosegerät zu fixieren und die Sterilisierungshülle mit dem Aufsetzteil an dem Therapie- und/oder Diagnosegerätes festzulegen.

Aus der US-Patentschrift US 7 122 032 B2 ist ein mit Operationsmikroskop einer Sterilumhüllung bekannt, bei dem vorgesehen ist, die Sterilumhüllung mit einem sterilisierbaren elektrischen Steckverbinder zu durchstoßen, wobei der elektrische Steckverbinder einen Flansch aufweist, der die Sterilumhüllung am Mikroskopgehäuse festlegt.

Aus der DE 102 15 808 B4 ist ein Röntgengerät bekannt, das an der Halterung für den C-Bogen eine Markenanordnung trägt, die mittels eines Lageerfassungssystems erfaßbar ist, wodurch die Lage und Position des Röntgengerätes im Raum bestimmbar ist.

Aus den deutschen Patentschriften DE 103 60 025 B4 und DE 101 39 329 B4 sind Röntgendiagnostikeinrichtungen bekannt, bei denen der Röntgenstrahlenempfänger eine Markenanordnung trägt.

Aus der deutschen Patentschrift DE 199 08 903 C2 ist eine Lokalisationseinheit für bild- und positionsgebende Geräte bekannt, die auf einer Basisplatte Marken und Sensoren zur Lageerfassung mit unterschiedlichen Lageerfassungssystemen aufweist.

Aus der deutschen Patentschrift DE 196 25 411 C2 ist eine medizinische Anlage mit einem lösbar angeordneten verstellbaren Handgriff bekannt, bei dem eine Sterilabdeckung vorgesehen ist.

Aus der Praxis der Verwendung von Lokalisationseinheiten für diagnostische Geräte mit Sterilabdeckfolien ist bekannt, daß die Lokalisationseinheiten durch die Folie hindurch schlecht von dem entsprechenden Lageerfassungssystem erfaßbar sind. Zwar versucht man in der Praxis, die im wesentlichen transparente Sterilabdeckfolie eng an die Lokalisationseinheiten anzulegen und in dieser Lage temporär zu fixieren, wie dies beispielsweise beim zweiten Gesichtspunkt der Erfindung der DE 196 40 993 A1 vorgesehen ist. Insbesondere bei Lokalisationseinheiten oder Marken mit kleinem Krümmungsradien kann dies zum Durchstoßen der Sterilabdeckfolie und damit zum Verlust der Sterilität führen. In den Fällen, in denen vorgesehen ist, die Lokalisationseinheit unter Zwischenlage der Sterilabdeckfolie am Gehäuse des diagnostischen Gerätes in der Betriebsstellung zu halten, sind die mechanischen Eigenschaften der Sterilabdeckung in der aktuellen Orientierung am Gerät (beispielsweise Dicke, Kompressibilität, Faltenwurf, Schweiß- oder Nahtkanten) bei der Bestimmung der Position und Lage der Lokalisationseinheit im Koordinatensystem des diagnostischen Gerätes zu berücksichtigen, was in der Praxis schwer möglich ist, weil beispielsweise die Faltenfreiheit der Sterilabdeckung im Klemmbereich schwer kontrollierbar ist.

Aufgabe der Erfindung ist es, eine Lokalisationseinheit für eine mit einer Sterilabdeckfolie abgedeckten Röntgendiagnostikeinrichtung zu schaffen, deren Funktion unabhängig von den Eigenschaften der Sterilabdeckfolie gewährleistet ist.

Die Aufgabe der Erfindung wird dadurch gelöst, daß die Lokalisationseinheit sterilisierbar ist und einen Adapter aufweist, der Mittel zur Durchstoßung der Sterilabdeckfolie und Mittel zum temporären Fixieren der Sterilabdeckfolie am Gehäuse aufweist. Der Adapter ist in vorbestimmten Aufnahmen lösbar am Gehäuse der Röntgendiagnostikeinrichtung gehalten und reproduzierbar zu diesem positionierbar.

Die Erfindung wird an Hand der Abbildungen näher erläutert.

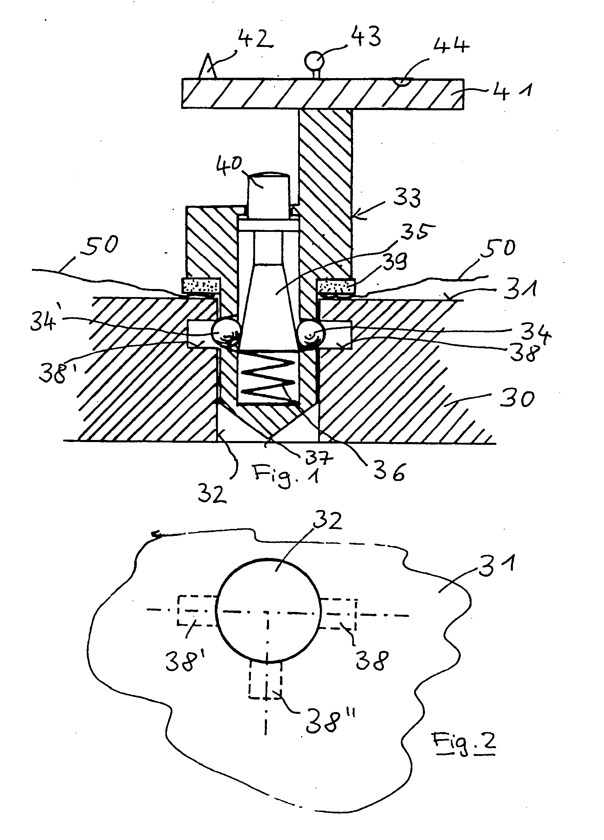

In Fig. 1 ist eine erfindungsgemäße Lokalisationseinheit mit einem Adapter (33) im Schnitt und in Fig. 2 in der Aufsicht schematisch dargestellt. Der Adapter (33) trägt an seiner Oberseite eine Halteplatte (41), die wiederum Marken unterschiedlicher Art (42, 43, 44) trägt. Die Marken sind von Lageerfassungssystemen unterschiedlicher Art erfaßbar und sind in einer vorbestimmten Anordnung bezüglich eines adaptereigenen Koordinatensystems angeordnet. Der Adapter weist einen unteren Teil auf, der von einer Aufnahmebohrung (32) aufgenommen wird und eine Spitze (37) trägt, vermittels der eine Sterilabdeckfolie (50) durchstoßen werden kann. In dem Gehäuse (30) der Röntgendiagnostikeinrichtung sind Bohrungen (38, 38', 38") vorgesehen, die mit konischen Bohrungen im Adapter (33) fluchten. Kugeln (34, 34', 34") werden durch eine Feder (36) und einen Konus (35) bezüglich der Konusachse radial nach außen gedrückt und verriegeln dadurch den Adapter (33) gegenüber dem Gehäuse (30). Der Konus (35) ist mit einem Druckknopf (40) verbunden, der bei Betätigung in Richtung der Spritze (37) ein radiales Spiel für die Kugeln (34, 34', 34") erzeugt, wodurch die Verriegelung zwischen Adapter (33) und Gehäuse (30) aufgehoben wird und der Adapter (33) vom Gehäuse (30) entfernt werden kann. Der Adapter (33) weist eine elastisch komprimierbare Dichtung (39) auf, die die Sterilabdeckfolie (50) temporär an das Gehäuse (30) andrückt und eine Verschiebung der Sterilabdeckfolie (50) gegenüber dem Gehäuse (30) verhindert.

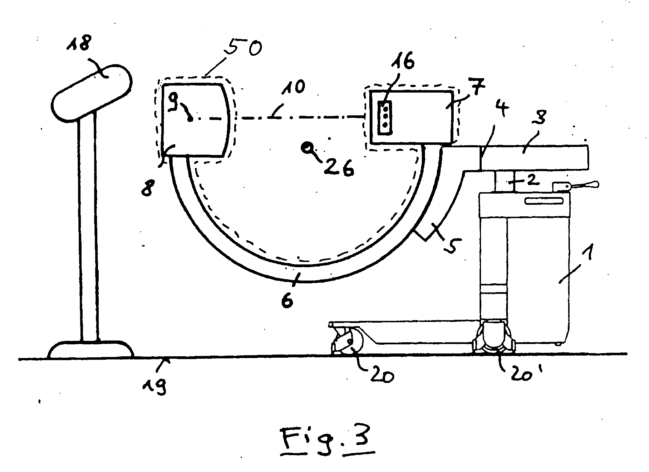

In Fig. 3 ist eine chirurgische Röntgendiagnostikeinrichtung mit einem C-Bogen (6) dargestellt, an dessen Enden eine Röntgenstrahlenquelle (8) und ein Röntgenstrahlenempfänger (7) angeordnet sind. Der C-Bogen ist an einer Halterung (5) mehrfach verstellbar gelagert. Die Röntgenstrahlenquelle (7), der C-Bogen (6) und der Röntgenstrahlenempfänger (7) sind mit einer vorzugsweise mehrstückigen Sterilabdeckfolie (50) abgedeckt. Am Röntgenstrahlenempfänger (7) ist eine Markenanordnung (16) vorgesehen, deren Lage mittels eines Lageerfassungssystems (18) erfaßbar sind.

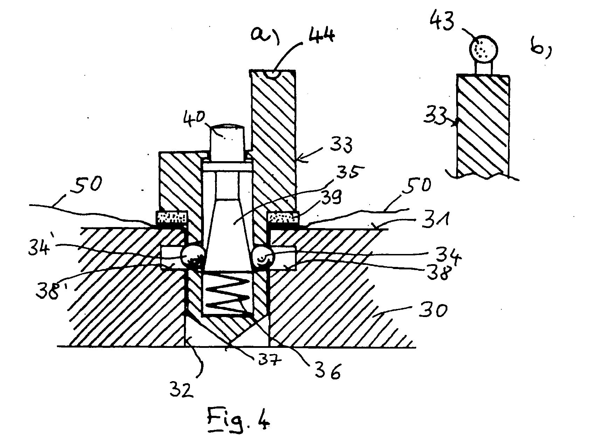

In Fig. 4 sind zwei weitere erfindungsgemäße Ausgestaltungen des Adapters (33) dargestellt. In Fig. 4a ist auf der Oberfläche des Adapters (33) eine Marke dritter Art (44) vorgesehen, die mit einem Pointer eines Lageerfassungssystems antastbar ist. In Fig. 4b ist am oberen Ende des Adapters (33) eine Marke der zweiten Art (43) vorgesehen. Hierbei kann es sich beispielsweise bei Verwendung eines optischen Lageerfassungssystems um eine Autoreflexionskugel oder um eine Lichtquelle, z.B. um eine LED-Lichtquelle handeln.

Es ist im Rahmen der Erfindung vorgesehen, daß die Marken (42, 43, 44) am Adapter (33) oder an der Halteplatte (41) lösbar gehalten sind.

In den Ausführungsbeispielen ist ein Haltemechanismus für den Adapter dargestellt, der durch Druckknopfbetätigung lösbar ist und durch Einrasten in eine entsprechende Aufnahme des Gehäuses reproduzierbar positioniert wird. Es ist im Rahmen der Erfindung auch vorgesehen, einen Adapter zu schaffen, der eine Marke (42, 43, 44) trägt und reproduzierbar in die Struktur des Gehäuses einschraubbar ist, wobei der Adapter Mittel zum Durchstoßen und zum temporären Fixieren der Sterilabdeckfolie am Gehäuse der Röntgendiagnostikeinrichtung aufweist. In diesem Fall würde der Adapter zunächst mit der Dichtung (39) über der Aufnahmebohrung aufgesetzt werden. Durch Eindrücken der Spitze (37) in die Folie wird diese durchstoßen, wobei durch die zusammengedrückte Dichtung (39) die Sterilabdeckfolie am Gehäuse (30) gehalten wird. Um beim Einschrauben des Adapters in ein vorgesehenes Gewinde im Gehäuse ein Verdrehen der Dichtung (39) zu verhindern, ist zwischen Dichtung (39) und Adapter (33) eine Scheibe vorgesehen. Es ist vorgesehen, die Scheibe in die Dichtung zu integrieren.

### Bezugszeichenliste:

- 1: Gerätewagen
- 2: Säule
- 3: Horizontalführung
- 4: Schwenklager
- 5: C-Bogenhalterung
- 6: C-Bogen
- 7: Röntgenstrahlenempfänger
- 8: Röntgenstrahlenquelle
- 9: Brennfleck
- 10: Zentralstrahl
- 16: Markenanordnung
- 18: Lageerfassungssystem
- 19: Fußboden
- 20, 20': Rolle
- 26: Mittelpunkt des C-Bogens
- 30: Gehäuse
- 31: Gehäuseoberfläche
- 32: Aufnahmebohrung
- 33: Adapter
- 34, 34', 34": Kugel
- 35: Konus
- 36: Feder
- 37: Spitze
- 38, 38', 38": Bohrung
- 39: Dichtung
- 40: Betätigungsknopf
- 41: Halteplatte
- 42: Marke
- 43: Marke
- 44: Marke
- 50: Sterilabdeckfolie

## Patentansprüche

1. Sterilisierbare Lokalisationseinheit zur lösbaren und reproduzierbar positionierbaren Anbringung am Gehäuse (30) einer mit einer Sterilabdeckfolie (50) abgedeckten nicht-sterilen chirurgischen Röntgendiagnostikeinrichtung mit einem Adapter (33) und wenigstens einer von einem Lageerfassungssystem (18) erfaßbaren Marke (42, 43, 44),
**dadurch gekennzeichnet, daß** der Adapter (33) ein Mittel zum Durchstoßen der Sterilabdeckfolie (50), ein Mittel zum lagegenauen Positionieren des Adapters (33) am Gehäuse (30) und ein Mittel zum temporären Fixieren der Sterilabdeckfolie (50) am Gehäuse (30) während und nach dem Anbringen des Adapters (33) am Gehäuse (30) aufweist, wobei das Mittel zum temporären Fixieren der Sterilabdeckfolie (50) am Gehäuse (30) aus einer elastisch verformbaren umlaufenden Dichtung (39) besteht, die beim Positionieren des Adapters (33) eine Kraft auf die Sterilabdeckfolie (50) in Richtung senkrecht zu der Oberfläche des Gehäuses (30) erzeugt.

2. Sterilisierbare Lokalisationseinheit nach Anspruch 1,
**dadurch gekennzeichnet, daß** das Mittel zur lagegenauen Positionierung des Adapters (33) aus einem mit der Druckkraft einer Feder (36) beaufschlagten Druckknopfmechanismus besteht, durch den mittels eines Konus (35) und Kugeln (34, 34') der Adapter (33) gegenüber dem Gehäuse (30) ver- und entriegelbar ist.

3. Sterilisierbare Lokalisationseinheit nach Anspruch 1,
**dadurch gekennzeichnet, daß** das Mittel zur lagegenauen Positionierung des Adapters (33) aus einem in das Gehäuse (30) einschraubbaren Gewinde und einem am Gehäuse (30) anschlagbaren Anschlagbund besteht.

4. Sterilisierbare Lokalisationseinheit nach einem der Ansprüche 1-3,
**dadurch gekennzeichnet, daß** der Adapter eine Halteplatte (41) aufweist, an der unterschiedliche Marken (42, 43, 44) in vorbestimmten Positionen bezüglich eines adaptereigenen Koordinatensystems angeordnet sind.

5. Sterilisierbare Lokalisationseinheit nach einem der Ansprüche 1-4,
**dadurch gekennzeichnet, daß** der Adapter wenigstens eine Marke (44) aufweist, die von einem Pointer des Lageerfassungssystems (18) antastbar ist.

6. Sterilisierbare Lokalisationseinheit nach einem der Ansprüche 1-4,
**dadurch gekennzeichnet, daß** der Adapter wenigstens eine Marke (43) aufweist, die eine von einem Lageerfassungssystem (18) erfaßbare Autoreflexionskugel ist.
